# EUROPEAN PATENT APPLICATION

(11) **EP 4 714 358 A1**
(43) Date of publication of application: **25.03.2026**
(21) Application number: 24806985.8
(22) Date of filing: 23.04.2024
(51) Int. Cl.: A61B 5/352, A61B 5/349, A61B 5/08

(54) **BIOLOGICAL INFORMATION MEASUREMENT METHOD, PROGRAM, AND ELECTROCARDIOGRAPH**

(30) Priority: 15.05.2023 JP 2023080023
(71) Applicant: SHIMADZU CORPORATION, Kyoto-shi, Kyoto 604-8511 (JP)
(72) Inventor: KITAGAWA, Akane, Kyoto-shi, Kyoto 604-8511 (JP); MURATA, Koichi, Kyoto-shi, Kyoto 604-8511 (JP); FURUTA, Masafumi, Kyoto-shi, Kyoto 604-8511 (JP)
(74) Representative: Müller-Boré & Partner Patentanwälte PartG mbB
(86) International application number: PCT/JP2024/015817
(87) International publication number: WO 2024/237031

(57) **Abstract**

A biological information measurement method includes a step of acquiring an electrocardiogram signal (S10); a step of specifying, in each heartbeat of the electrocardiogram signal, a target region including a peak of an R-wave and a peak of an S-wave (S14); a step of specifying, in the target region of each heartbeat, a position of the peak of the S-wave (S18); a step of specifying, in the target region of each heartbeat, a position of a peak that is temporally prior to the position of the peak of the S-wave as a position of the peak of the R-wave (S20); a step of specifying a QRS interval within the target region of each heartbeat, using the position of the peak of the R-wave and the position of the peak of the S-wave (S22); and a step of calculating a heart rate by using intervals between the positions of the R-wave peaks specified in each heartbeat of the electrocardiogram signal (S30).

## Description

### TECHNICAL FIELD

The present invention relates to the measurement of biological information using an electrocardiogram waveform, and particularly relates to the estimation of heart rate and respiration rate using an electrocardiogram waveform.

### BACKGROUND ART

Electrocardiac potential and respiratory information are attracting attention as biological information used for the evaluation of autonomic nervous system function.

Regarding electrocardiac potential, Non-Patent Literature 1 (myBeat ~Interface of Mind and Body~, [online], UNION TOOL CO., [searched on February 7, 2023], Internet <URL: https://www.uniontool-mybeat.com/> discloses a wearable electrocardiograph.

Regarding respiratory information, Non-Patent Literature 2 (ResMo, [online], Aimedic MMT Co., Ltd., [searched on February 7, 2023], Internet <URL: https://www.aimedicmmt.co.jp/product/ResMo/> discloses a device that measures respiration rate. Furthermore, Non-Patent Literature 3 ("[Press Release] Your Breathing Analyzed by AI _ Development of "ZenTracker (tentative name)," a Smart Device to Visualize Breathing Rhythm. Presented as a Reference Exhibit at the Next-Generation Healthcare Project 2021.", [online], Koto Co., Ltd., [searched on February 7, 2023], Internet <URL: https://www.koto.co.jp/2021/11/22/zentracker/> discloses a device that measures information such as breathing depth and respiration rate.

Regarding devices that handle such biological information (electrocardiac potential and respiratory information), Non-Patent Literature 4 (Polymate Pro MP6000, [online], Miyuki Giken Co., Ltd., [searched on February 7, 2023], Internet <URL: https://www.miyuki-net.co.jp/jp/polymate/MP6000/> discloses a biological signal recording device that supports input from multiple types of measurement sensors, including electrocardiogram and respiratory information.

For the collection of multiple types of biological information, a subject may be required to wear a measurement device for each type of biological information, which can be a significant burden. In this regard, Patent Literature 1 (Japanese Unexamined Patent Application Publication No. 2016-083369) discloses a technique for acquiring respiratory information from electrocardiac potential. This technique extracts electromyogram signals representing different directional components of muscle action potential changes accompanying respiratory movements from the electrocardiac potential, and synthesizes them to generate a respiratory signal. This technique requires the measurement of at least two electrocardiogram signals representing changes in cardiac electromotive force in mutually different directions.

On the other hand, Non-Patent Literature 5 (Toshio Watanabe, Shigeru Kagaya, Mitsuhiro Miyazawa, Isao Kubota, Kozue Ikeda, Michiyasu Yamaki, Shoji Yasui, Electrocardiology, "On a Method for Estimating Respiration Rate from Electrocardiogram Time-Series Data," Japan, Japanese Heart Rhythm Society, July 30, 1987, Vol. 7, No. 3, pp. 287-293) proposes a method for estimating respiration rate using R-R interval and QRS amplitude time-series data of an electrocardiogram.

Furthermore, Non-Patent Literature 6 (JIAPU PAN, WILLIS J. TOMPKINS, "A Real-Time QRS Detection Algorithm", [online], March 1985, Institute of Electrical and Electronics Engineers) discloses an example of a method for detecting the R-wave peak in an electrocardiogram.

### CITATION LIST

### PATENT LITERATURE

[Patent Literature 1] Japanese Unexamined Patent Application Publication No. 2016-083369

### NON-PATENT LITERATURE

[Non-Patent Literature 1] "myBeat ~Interface of Mind and Body~", [online], UNION TOOL CO., [searched on February 7, 2023], Internet < URL:https://www.uniontool-mybeat.com/>
[Non-Patent Literature 2] "ResMo", [online], Aimedic MMT Co., Ltd., [searched on February 7, 2023], Internet < URL:https://www.aimedicmmt.co.jp/product/ResMo/>
[Non-Patent Literature 3] "[Press Release] 'Your Breathing Analyzed by AI' _ Development of 'ZenTracker (tentative name),' a Smart Device to Visualize Breathing Rhythm. Presented as a Reference Exhibit at the Next-Generation Healthcare Project 2021.", [online], Koto Co., Ltd., [searched on February 7, 2023], Internet <URL:https://www.koto.co.jp/2021/11/22/zentracker/>
[Non-Patent Literature 4] "Polymate Pro MP6000", [online], Miyuki Giken Co., Ltd., [searched on February 7, 2023], Internet < URL: https://www.miyukinet.co.jp/jp/polymate/MP6000/>
[Non-Patent Literature 5] Toshio Watanabe, Shigeru Kagaya, Mitsuhiro Miyazawa, Isao Kubota, Kozue Ikeda, Michiyasu Yamaki, Shoji Yasui, Electrocardiology, "On a Method for Estimating Respiration Rate from Electrocardiogram Time-Series Data," Japan, Japanese Heart Rhythm Society, July 30, 1987, Vol. 7, No. 3, pp. 287-293
[Non-Patent Literature 6] JIAPU PAN, WILLIS J. TOMPKINS, "A Real-Time QRS Detection Algorithm", [online], March 1985, Institute of Electrical and Electronics Engineers

### SUMMARY OF INVENTION

### TECHNICAL PROBLEM

Non-Patent Literature 5 requires the detection of the R-wave peak to acquire the R-R interval and QRS amplitude value of the electrocardiogram. Depending on the electrode placement in the measurement of the electrocardiogram waveform, the R-wave peak may not be detected with sufficient accuracy.

The present invention has been conceived in view of such circumstances, and an object thereof is to provide a technique for detecting an R-wave peak and appropriately estimating a respiration rate, regardless of the shape of the QRS waveform, which depends on the placement of the measurement electrodes for the electrocardiogram in the electrocardiogram waveform.

### SOLUTION TO PROBLEM

A biological information measurement method according to an aspect of the present disclosure includes a step of acquiring an electrocardiogram signal from the output of a first electrode and a second electrode attached to a measurement subject; a step of specifying, in each heartbeat of the electrocardiogram signal, a target region including a peak of an R-wave and a peak of an S-wave; a step of specifying, in the target region of each heartbeat, a position of the peak of the S-wave; a step of specifying, in the target region of each heartbeat, a position of a peak that is temporally prior to the position of the peak of the S-wave as a position of the peak of the R-wave; a step of specifying a QRS interval within the target region of each heartbeat, using the position of the peak of the R-wave and the position of the peak of the S-wave; a step of calculating a heart rate of the measurement subject by using intervals between the positions of the R-wave peaks specified in each heartbeat of the electrocardiogram signal; and a step of calculating a respiration rate of the measurement subject by using a time series of amplitude in a derivative electrocardiogram of the QRS interval specified in each heartbeat of the electrocardiogram signal, and the heart rate.

### ADVANTAGEOUS EFFECTS OF INVENTION

According to the present disclosure, a technique is provided for enabling the estimation of a respiration rate even when using an electrocardiogram waveform with a QRS shape that makes it difficult to directly detect the R-wave peak.

### BRIEF DESCRIPTION OF DRAWINGS

[FIG. 1] FIG. 1 is an external view of an electrocardiograph 100 according to an embodiment of the present disclosure.
[FIG. 2] FIG. 2 is a side view of a main body device 1.
[FIG. 3] FIG. 3 is a rear view of the main body device 1.
[FIG. 4] FIG. 4 is a diagram for explaining an example of a method of using the electrocardiograph 100.
[FIG. 5] FIG. 5 is a diagram showing a hardware configuration of the electrocardiograph 100.
[FIG. 6] FIG. 6 is a diagram showing a specific example of an electrocardiogram signal.
[FIG. 7] FIG. 7 is a diagram showing a specific example of a target region specified in the electrocardiogram signal.
[FIG. 8] FIG. 8 is a diagram showing an example of an S-wave peak position, an R-wave peak position, and a QRS interval specified in a target region.
[FIG. 9] FIG. 9 is a flowchart of processing performed for the measurement of heart rate and respiration rate in the electrocardiograph 100.
[FIG. 10] FIG. 10 is a diagram showing a specific example of the estimation results by the method according to the present embodiment for respiration rates from two types of electrode placements.

### DESCRIPTION OF EMBODIMENTS

Hereinafter, embodiments of the present disclosure will be described in detail with reference to the drawings. Note that the same or corresponding parts in the drawings are denoted by the same reference signs, and a description thereof will not be repeated.

### [Appearance of Electrocardiograph]

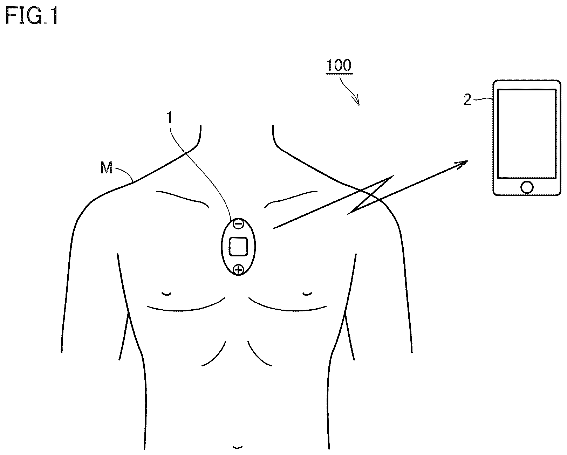

FIG. 1 is an external view of an electrocardiograph 100 according to an embodiment of the present disclosure. The electrocardiograph 100 includes a main body device 1 attached to the body of a measurement subject M, and a processing device 2 connected to the main body device 1 so as to be communicable wirelessly or by wire. The processing device 2 may be incorporated into the main body device 1. The measurement subject M is a human, and is an example of a measurement subject. The measurement subject may be a living organism other than a human, such as an animal.

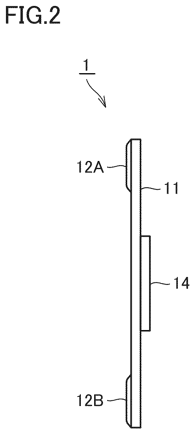

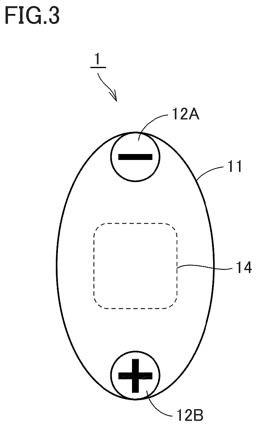

FIG. 2 is a side view of the main body device 1. FIG. 3 is a rear view of the main body device 1. The main body device 1 includes a holder 11, electrodes 12A and 12B, and a measurement circuit 14. The electrode 12A is a Negative electrode, and the electrode 12B is a Positive electrode. The electrodes 12A and 12B and the measurement circuit 14 are fixed to the holder 11. In one implementation, the holder 11 is configured by a bendable sheet-like material. However, the form of the holder 11 is not limited to that shown in FIG. 2 and FIG. 3. The holder 11 may have a case-like form that accommodates the electrodes 12A and 12B and/or the measurement circuit 14. In one implementation, the electrodes 12A and 12B have the form of circular metal pads with conductivity, and are attached to one surface of the holder 11 at a predetermined distance from each other.

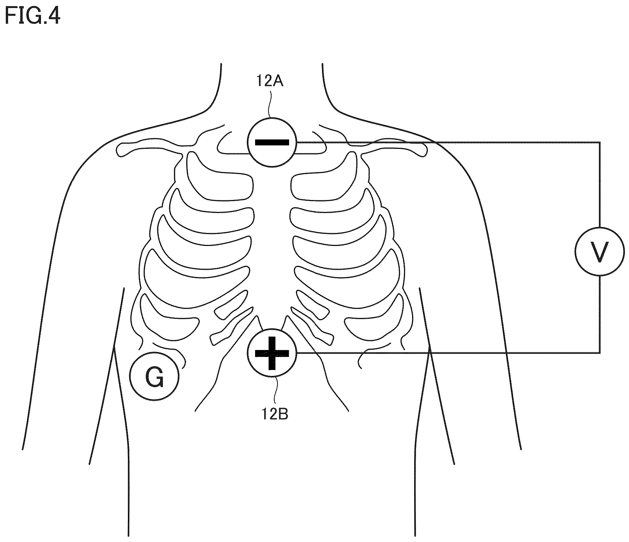

FIG. 4 is a diagram for explaining an example of a method of using the electrocardiograph 100. In the example of FIG. 4, the electrode 12A is attached to the upper side of the sternum of the measurement subject, and the electrode 12B is attached to the xiphoid process of the measurement subject. The measurement circuit 14 acquires an electrocardiogram signal from the output of the electrode 12A and the electrode 12B.

### [Hardware Configuration]

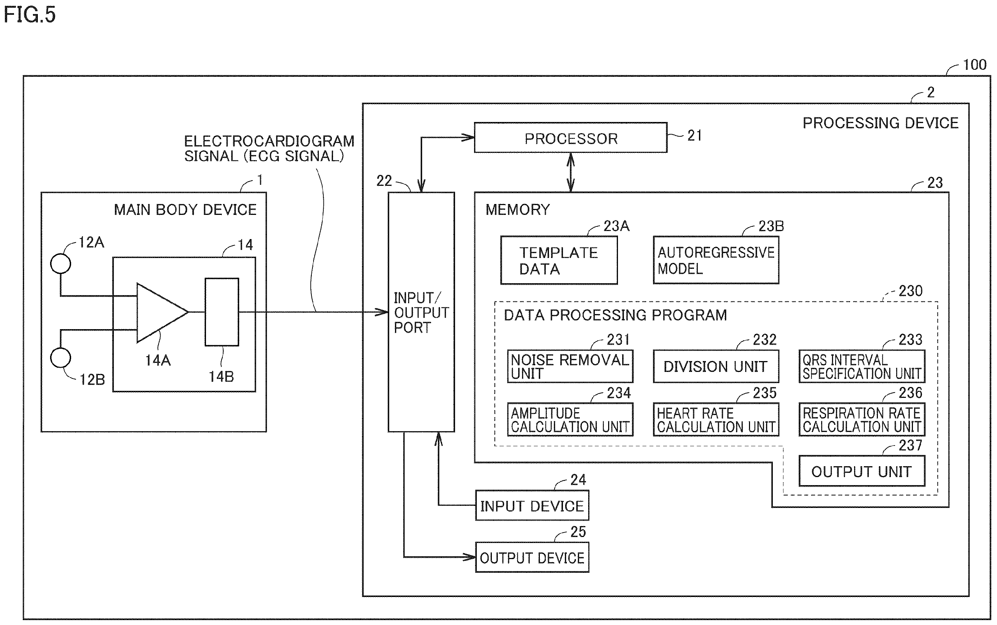

FIG. 5 is a diagram showing a hardware configuration of the electrocardiograph 100. The hardware configurations of the main body device 1 and the processing device 2 will be described with reference to FIG. 5.

### (Main Body Device 1)

In the main body device 1, the electrodes 12A and 12B are connected to the measurement circuit 14. The measurement circuit 14 is composed of analog and digital electric circuits. The measurement circuit 14 includes an electrocardiogram signal detection unit 14A and a transmission unit 14B.

The electrocardiogram signal detection unit 14A measures the potential difference between the electrode 12A and the electrode 12B, and generates an electrocardiogram signal by applying various processes to the measurement result. The applied process is, for example, noise cutting from a signal representing the above-mentioned potential difference. The transmission unit 14B is a communication interface and transmits the electrocardiogram signal generated by the electrocardiogram signal detection unit 14A to the processing device 2.

### (Processing Device 2)

The processing device 2 may be configured by a general-purpose computer such as a smartphone, a tablet terminal, or a desktop computer.

The processing device 2 includes a processor 21, an input/output port 22, a memory 23, an input device 24, and an output device 25.

The processor 21 is an example of an electric circuit, and controls the operation of the processing device 2 by executing a given program. The program executed by the processor 21 may be stored in the memory 23, or may be stored in a storage device external to the processing device 2.

The memory 23 stores data in a non-transitory manner. The data stored in the memory 23 includes template data 23A, an autoregressive model 23B, and a data processing program 230. The template data 23A includes one or more waveform templates of an electrocardiogram signal for a certain period of time including an R-wave peak.

The autoregressive model 23B is configured to output a frequency of respiration relative to a heartbeat by receiving a time series of the amplitude of the QRS interval in the electrocardiogram signal. The autoregressive model 23B may be realized as a machine learning model, or may be realized as a rule-based algorithm. The rule-based algorithm is, for example, an algorithm that realizes a method of obtaining frequency components using a Fourier transform.

The data processing program 230 is a program for calculating the heart rate and respiration rate from the electrocardiogram signal. The data processing program 230 may be stored in the memory 23, or may be stored in an external storage device accessible by the processor 21.

The data processing program 230, when executed by the processor 21, causes the processor 21 to include at least seven functions: a noise removal unit 231, a division unit 232, a QRS interval specification unit 233, an amplitude calculation unit 234, a heart rate calculation unit 235, a respiration rate calculation unit 236, and an output unit 237. A description of each function will be given later.

The input device 24 accepts the input of information to the processing device 2, and is configured by, for example, software buttons and/or physical buttons.

In one implementation, the input device 24 includes a measurement start button and a measurement end button. When the measurement start button is operated, the processor 21 instructs the main body device 1 to start transmitting the electrocardiogram signal. In response to this, the transmission unit 14B starts transmitting the electrocardiogram signal to the processing device 2. When the measurement end button is operated, the processor 21 instructs the main body device 1 to end the transmission of the electrocardiogram signal. In response to this, the transmission unit 14B ends the transmission of the electrocardiogram signal to the processing device 2. The processor 21 may calculate the heart rate and the respiration rate in response to the operation of the measurement end button, and output the result to the output device 25.

The calculation of the heart rate and the respiration rate does not have to be performed immediately after the end of the measurement. More specifically, in response to the operation of the measurement end button, the processor 21 may store the electrocardiogram signal transmitted from the main body device 1 in the memory 23. The input device 24 may include an analysis button. When the analysis button is operated, the processor 21 may use the electrocardiogram signal stored in the memory 23 to calculate the heart rate and the respiration rate, and output the result to the output device 25.

The processor 21 may accept a designation of a range to be subjected to the calculation of the heart rate and/or the respiration rate for the electrocardiogram signal stored in the memory 23. For example, when an electrocardiogram signal for 10 seconds is stored in the memory 23, the processor 21 may accept a designation to subject only the electrocardiogram signal for the first 5 seconds to the calculation of the heart rate and/or the respiration rate. In such a case, the processor 21 may calculate the heart rate and/or the respiration rate using only the designated portion of the electrocardiogram signal.

The output device 25 outputs information in accordance with an instruction from the processor 21, and is configured by, for example, a display and/or a speaker.

### [Noise Removal]

Noise removal by the noise removal unit 231 will be described. The noise removal unit 231 removes noise from the electrocardiogram signal. For noise removal, for example, an 8-16 Hz IIR zero-phase filter is used.

### [Specification of Target Region]

Specification of a target region by the division unit 232 will be described. The division unit 232 specifies, from the electrocardiogram signal by template matching, a target region including the position of the R-wave peak of a heartbeat. For the template matching, a template stored as the template data 23A is used. As the target region, for example, a signal corresponding to a time of about 150 ms is specified.

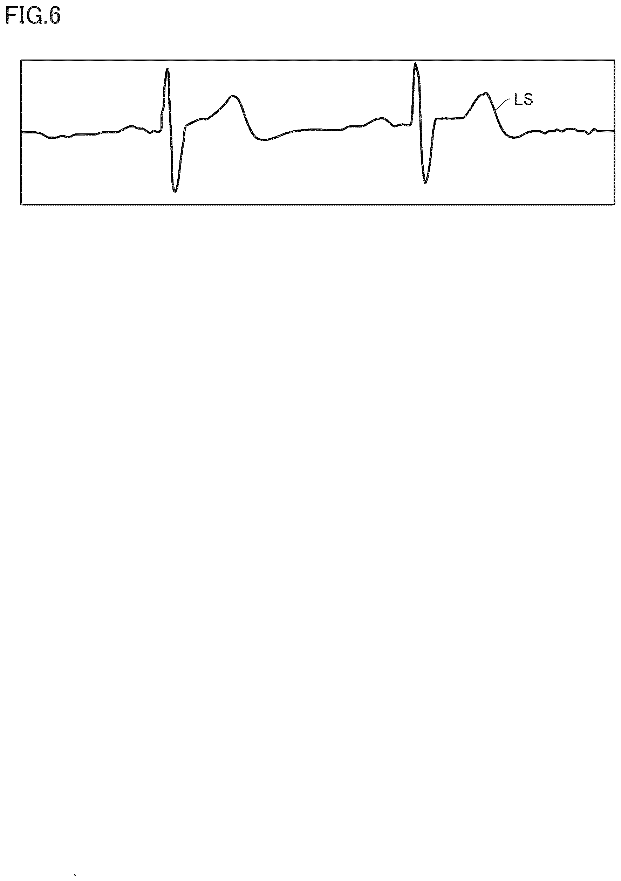

FIG. 6 is a diagram showing a specific example of an electrocardiogram signal. In FIG. 6, a waveform LS of the electrocardiogram signal is shown. By template matching, a target region including the position of the R-wave peak of each heartbeat is specified from the waveform LS.

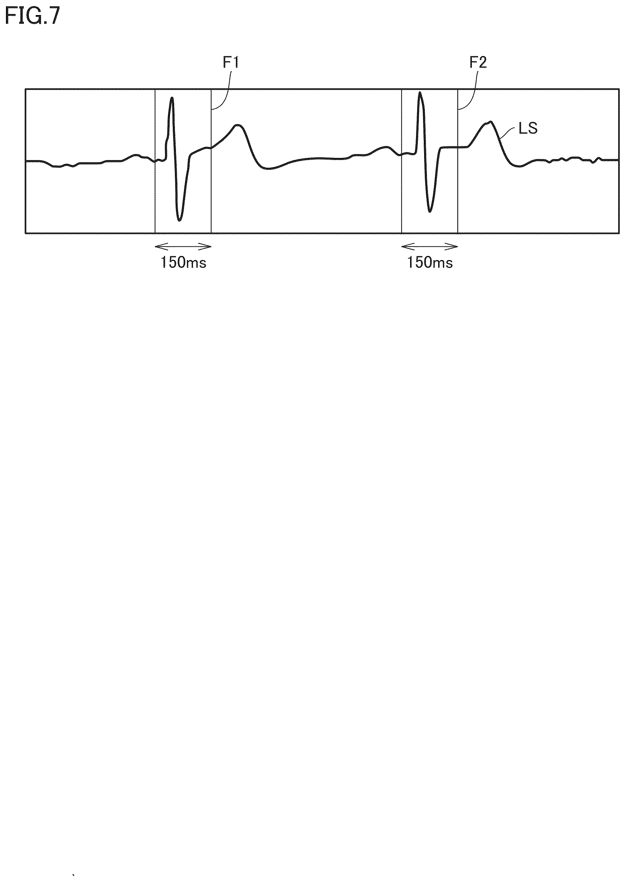

FIG. 7 is a diagram showing a specific example of a target region specified in the electrocardiogram signal. In FIG. 7, two frames F1 and F2 representing target regions are shown. In the example of FIG. 7, two target regions (a portion in the frame F1 and a portion in the frame F2) are specified from the waveform LS.

### [Specification of S-wave and R-wave Peak Positions and QRS Interval]

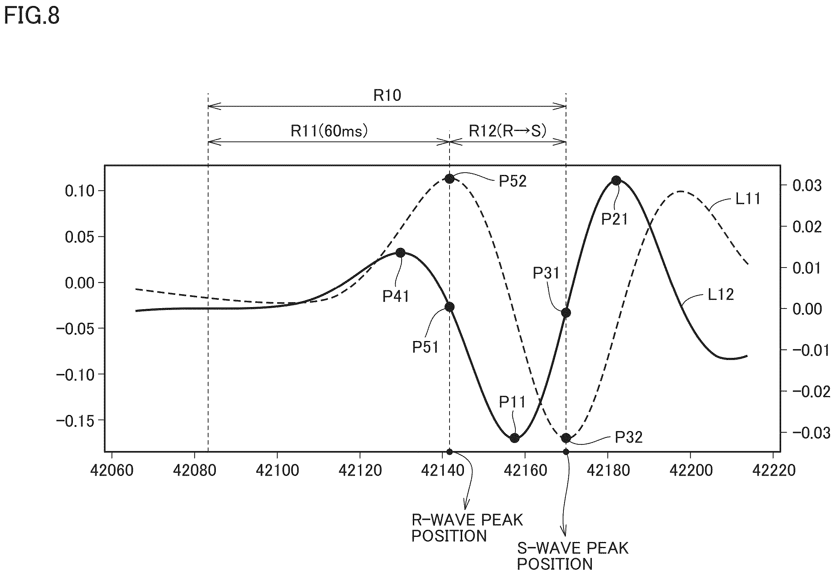

Specification of an S-wave peak position, an R-wave peak position, and a QRS interval by the QRS interval specification unit 233 will be described with reference to FIG. 8. FIG. 8 is a diagram showing an example of an S-wave peak position, an R-wave peak position, and a QRS interval specified in a target region.

First, the QRS interval specification unit 233 specifies the position of the S-wave peak in the target region. More specifically, the QRS interval specification unit 233 generates a derivative waveform of each target region specified as described above, and specifies a minimum value in the derivative waveform. In FIG. 8, a dashed line L11 represents the target region (electrocardiogram signal), and a solid line L12 represents the derivative waveform. The left vertical axis represents numerical values of the electrocardiogram signal, and the right vertical axis represents numerical values of the derivative waveform. The horizontal axis represents measurement time. In FIG. 8, the minimum value of the derivative waveform is indicated by a point P11.

Then, the QRS interval specification unit 233 specifies a maximum value (first maximum value) in a section after the minimum value in the target region (a section corresponding to a time later than the minimum value). In FIG. 8, the first maximum value is indicated by a point P21.

Then, the QRS interval specification unit 233 specifies, in the above-mentioned section, a location closest to zero between the minimum value and the first maximum value as a point corresponding to the position of the S-wave peak. In FIG. 8, the point corresponding to the position of the S-wave peak is indicated by a point P31 on the solid line L12 (derivative waveform), and by a point P32 on the dashed line L11 (electrocardiogram signal). That is, the point P32 is specified as the position of the S-wave peak.

Next, the QRS interval specification unit 233 specifies the position of the R-wave peak. More specifically, it specifies a maximum value (second maximum value) in a section before the minimum value in the target region (a section corresponding to a time earlier than the minimum value). In FIG. 8, the second maximum value is indicated by a point P41.

Then, the QRS interval specification unit 233 specifies, in the above-mentioned section, a location closest to zero between the minimum value and the second maximum value as a point corresponding to the position of the R-wave peak. In FIG. 8, the point corresponding to the position of the R-wave peak is indicated by a point P51 on the solid line L12 (derivative waveform), and by a point P52 on the dashed line L11 (electrocardiogram signal). That is, the point P52 is specified as the position of the R-wave peak.

From the above, the QRS interval specification unit 233 specifies the minimum value of the derivative waveform of the electrocardiogram waveform in the target section, specifies the position of the S-wave peak as a point after the minimum value, and then specifies the position of the R-wave peak as a point before the minimum value. Thereby, the position of the R-wave peak is specified to be located temporally before the position of the S-wave peak.

Next, the QRS interval specification unit 233 specifies a QRS interval. More specifically, it specifies, of the target region, a section from 60 ms before the position of the R-wave peak to the position of the S-wave peak as the QRS interval. In FIG. 8, the QRS interval is shown as an interval R10. Note that an interval R11 represents a section from the position of the R-wave peak to 60 ms before it. An interval R12 represents a section from the position of the R-wave peak to the position of the S-wave peak.

### [Amplitude Calculation]

Calculation of amplitude by the amplitude calculation unit 234 will be described. The amplitude calculation unit 234 performs linear interpolation on the waveform of the electrocardiogram signal of the QRS interval, and generates a derivative waveform of the linearly interpolated waveform. Then, the amplitude calculation unit 234 calculates the difference between the maximum value and the minimum value of the generated derivative waveform as the amplitude. For example, if the maximum value is 0.5 and the minimum value is -0.3, the difference between the maximum value and the minimum value is 0.8 (0.5 - (-0.3)). The amplitude calculated by the amplitude calculation unit 234 means the amplitude in the derivative waveform of the electrocardiogram signal, not the amplitude in the electrocardiogram signal.

### [Heart Rate Calculation]

Calculation of the heart rate by the heart rate calculation unit 235 will be described. The heart rate calculation unit 235 calculates an average value of the intervals between adjacent R-wave peak positions, using the R-wave peak position of each target region specified by the QRS interval specification unit 233. Then, the QRS interval specification unit 233 calculates the heart rate using the above-mentioned average value. For example, if the average value of the intervals between R-wave peak positions is 2.0 seconds, the heart rate is calculated as 30 beats/minute (60 ÷ 2).

### [Respiration Rate Calculation]

Calculation of the respiration rate by the respiration rate calculation unit 236 will be described. The respiration rate calculation unit 236 acquires the amplitude of each target region calculated by the amplitude calculation unit 234 as a time series of amplitude. Then, the respiration rate calculation unit 236 acquires the frequency of the time series of amplitude by applying the time series of amplitude as a point process to the autoregressive model 23B.

Then, the respiration rate calculation unit 236 calculates the respiration rate as the product of the frequency of the time series of amplitude and the heart rate calculated by the heart rate calculation unit 235.

### [Generation of Information for Output]

Generation of information for output by the output unit 237 will be described. In one implementation, the output unit 237 generates screen information for displaying the heart rate and the respiration rate calculated as described above, and transmits it to the output device 25. Thereby, the output device 25 displays a screen representing the heart rate and the respiration rate. The heart rate and the respiration rate may be displayed as estimated values.

### [Flow of Processing]

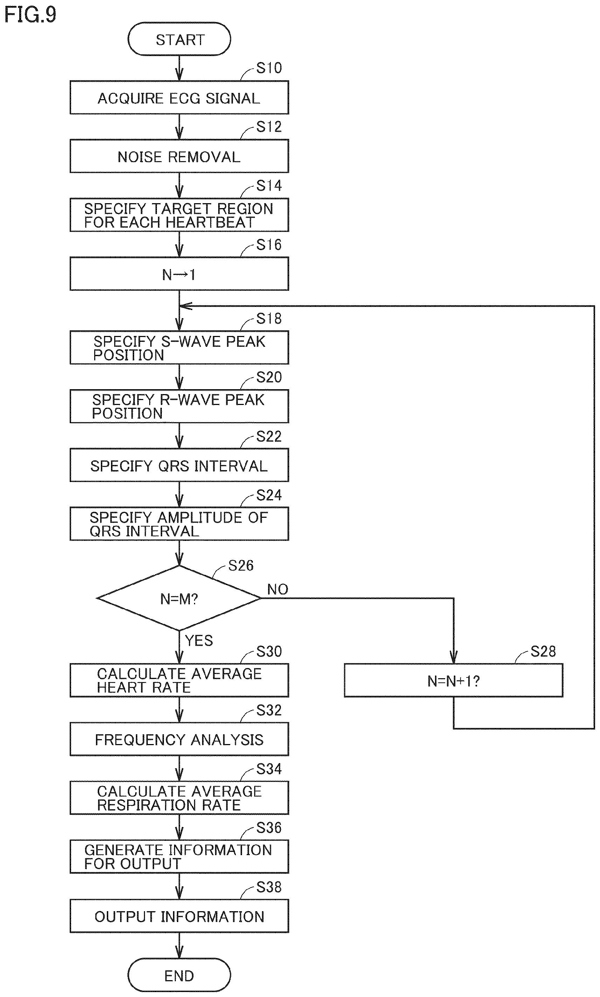

FIG. 9 is a flowchart of processing performed for the measurement of heart rate and respiration rate in the electrocardiograph 100. In one implementation, the electrocardiograph 100 performs the processing of FIG. 9 by the processor 21 executing a given program. In one implementation, the electrocardiograph 100 starts the processing of FIG. 9 when an operation to instruct the end of measurement of the electrocardiogram signal is performed on the input device 24, or in response to an operation to instruct the start of measurement of the heart rate and the respiration rate being performed.

In step S10, the electrocardiograph 100 acquires an electrocardiogram signal.

In step S12, the electrocardiograph 100 removes noise from the electrocardiogram signal, as described above, for example, as a function of the noise removal unit 231.

In step S14, the electrocardiograph 100 specifies a target region of each heartbeat in the electrocardiogram signal acquired in step S10, for example, as a function of the division unit 232 as described above, and then assigns an ID to each specified target region. When M target regions (target regions for M heartbeats) are specified from the electrocardiogram signal, an ID of a non-overlapping numerical value from 1 to M is assigned to each of the M target regions.

In step S16, the electrocardiograph 100 sets the value of a variable N used in the processing of FIG. 9 to 1.

In steps S18 to S22, the electrocardiograph 100, for the N-th target region, specifies the S-wave peak position, the R-wave peak position, and the QRS interval, as described above as a function of the QRS interval specification unit 233.

In step S24, the electrocardiograph 100, for the N-th target region, calculates the amplitude of the QRS interval, for example, as a function of the amplitude calculation unit 234 as described above.

In step S26, the electrocardiograph 100 determines whether or not the value of the variable N has reached the above-mentioned M. If the electrocardiograph 100 determines that the value of the variable N has reached the above-mentioned M (YES in step S26), it proceeds with the control to step S30, and if not (NO in step S26), it proceeds with the control to step S28.

In step S28, the electrocardiograph 100 increments the value of the variable N by 1 and returns the control to step S18.

In step S30, the electrocardiograph 100 calculates the average heart rate of the measurement subject, for example, as a function of the heart rate calculation unit 235 as described above. For the calculation of the average heart rate, the R-wave peak positions specified as described above are used. Note that, for the R-wave peak positions specified for each of the M target regions, a selection may be made as to which are used and which are not used for the calculation of the average heart rate, according to a given condition.

In step S32, the electrocardiograph 100 calculates the frequency of the QRS amplitude by frequency analysis of the time series of the QRS amplitude of the electrocardiogram signal, for example, as a function of the respiration rate calculation unit 236 as described above.

In step S34, the electrocardiograph 100 calculates the average respiration rate of the measurement subject, for example, as a function of the respiration rate calculation unit 236 as described above.

In step S36, the electrocardiograph 100 generates information for outputting the calculation results (output information) for the average heart rate and/or the average respiration rate calculated as described above.

An example of the output information is screen information for displaying the values of the average heart rate and the average respiration rate. Another example is audio information of these values. Still another example is screen information for displaying, together with these values, the state of the measurement subject specified from these values (for example, "relaxed state").

In step S38, the electrocardiograph 100 outputs the output information generated in step S36 to the output device 25 (or an external output device). Thereafter, the electrocardiograph 100 ends the processing of FIG. 9.

In the present embodiment described above, for the calculation of the heart rate and the respiration rate, the measurement subject wears only the main body device 1. That is, what the measurement subject wears is one small wearable measuring instrument. Therefore, the heart rate and the respiration rate of the measurement subject can be measured simultaneously without restricting the movement of the measurement subject and with a reduced burden of wearing.

Furthermore, since the main body device 1 is attached at a position on the sternum of the measurement subject, it is less susceptible to the influence of myoelectric noise generated by the movement of the measurement subject's arms, etc., in the measurement of the electrocardiac potential and respiration rate of the measurement subject.

Furthermore, since the measurement method in the present embodiment can be applied to a characteristic electrocardiogram waveform with a large S-wave, it can be applied to more electrode attachment placements. More specifically, the measurement method in the present embodiment enables the estimation of the respiration rate using an electrocardiogram signal even when applied to many placements, such as not only the NASA lead placement but also the CM5 lead placement.

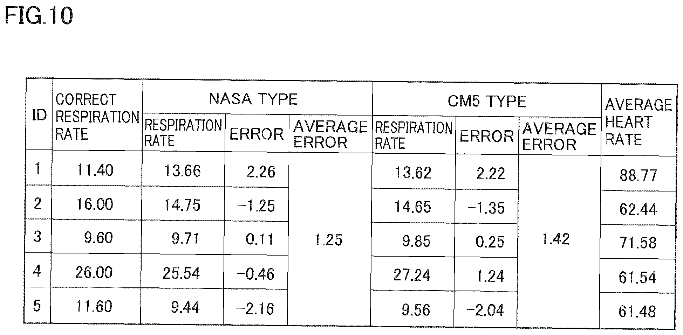

FIG. 10 is a diagram showing a specific example of the estimation results by the method according to the present embodiment for respiration rates from two types of electrode placements. In FIG. 10, "NASA" represents a value related to the respiration rate using an electrocardiogram signal obtained with a NASA lead type electrode placement. In the NASA lead type electrode placement, the electrode is positioned near the upper end of the sternum. "CM5" represents a value related to the respiration rate using an electrocardiogram signal obtained with a CM5 lead type electrode placement.

More specifically, FIG. 10 shows, for each of five phases identified by ID = 1 to 5, the correct respiration rate, the respiration rate obtained from the electrocardiogram signal obtained with the NASA lead type electrode placement, and the respiration rate obtained from the electrocardiogram signal obtained with the CM5 lead type electrode placement. The correct respiration rate means a respiration rate obtained by a method other than the electrocardiogram signal (for example, visual observation by a doctor). In FIG. 10, to explain the situation of each phase, the average heart rate specified for each phase is shown.

For example, in the phase with ID = 1, the correct respiration rate was 11.40. Furthermore, the respiration rate obtained from the electrocardiogram signal obtained with the NASA lead type electrode placement was 13.66, and the error with respect to the correct respiration rate was 2.26. Furthermore, the respiration rate obtained from the electrocardiogram signal obtained with the CM5 lead type electrode placement was 13.62, and the error with respect to the correct respiration rate was 2.22.

In FIG. 10, the "Average Error" for each of "NASA" and "CM5" represents the average value of the absolute values of the errors for ID = 1 to 5. In the example of FIG. 10, the average error of 1.25 for "NASA" is a value close to the average error of 1.42 for "CM5". From this, it can be said that according to the present embodiment, an estimated value of the respiration rate can be derived regardless of the type of electrode placement at the time of acquiring the electrocardiogram waveform.

### [Aspects]

It is understood by those skilled in the art that the plurality of exemplary embodiments described above are specific examples of the following aspects.

### (First Aspect)

A biological information measurement method according to one aspect may comprise: a step of acquiring an electrocardiogram signal from an output of a first electrode and a second electrode attached to a measurement subject; a step of specifying, in each heartbeat of the electrocardiogram signal, a target region including a peak of an R-wave and a peak of an S-wave; a step of specifying, in the target region of each heartbeat, a position of the peak of the S-wave; a step of specifying, in the target region of each heartbeat, a position of a peak that is temporally prior to the position of the peak of the S-wave as a position of the peak of the R-wave; a step of specifying a QRS interval within the target region of each heartbeat, using the position of the peak of the R-wave and the position of the peak of the S-wave; a step of calculating a heart rate of the measurement subject by using intervals between the positions of the R-wave peaks specified in each heartbeat of the electrocardiogram signal; and a step of calculating a respiration rate of the measurement subject by using a time series of amplitude in a derivative electrocardiogram of the QRS interval specified in each heartbeat of the electrocardiogram signal, and the heart rate.

According to the biological information measurement method described in the first aspect, a technique is provided for enabling the estimation of a respiration rate even when using an electrocardiogram waveform with a QRS shape that makes it difficult to directly detect the R-wave peak.

(Second Aspect) In the biological information measurement method according to the first aspect or the second aspect, the first electrode may be attached to an upper side of a sternum of the measurement subject, and the second electrode may be attached to a xiphoid process of the measurement subject.

According to the biological information measurement method described in the second aspect, a technique is provided for enabling the estimation of a respiration rate even when the electrocardiogram waveform is measured with an electrode placement where it is assumed that the QRS shape makes it difficult to directly detect the R-wave peak.

(Third Aspect) In the biological information measurement method according to the first aspect or the second aspect, the step of specifying the target region may include extracting the target region of each heartbeat by template matching with respect to the electrocardiogram signal.

According to the biological information measurement method described in the third aspect, a target region including the position of the R-wave peak is reliably extracted as the target region.

(Fourth Aspect) In the biological information measurement method according to any one of the first to third aspects, the step of specifying the position of the peak of the S-wave may include: obtaining a minimum value of a derivative waveform of the target region of each heartbeat; obtaining a first maximum value in a section located after the minimum value in the derivative waveform of the target region of each heartbeat; and extracting, as the position of the peak of the S-wave, a location where a value closest to 0 between the minimum value and the first maximum value is located in the derivative waveform of the target region of each heartbeat.

According to the biological information measurement method described in the fourth aspect, the position of the S-wave peak is specified more accurately in each target region.

(Fifth Aspect) In the biological information measurement method according to the fourth aspect, the step of specifying the position of the peak of the R-wave may include: obtaining a second maximum value in a section located temporally prior to the minimum value in the derivative waveform of the target region of each heartbeat; and extracting, as the position of the peak of the R-wave, a location where a value closest to 0 between the minimum value and the second maximum value is located in the derivative waveform of the target region of each heartbeat.

According to the biological information measurement method described in the fifth aspect, the position of the R-wave peak is specified more accurately in each target region.

(Sixth Aspect) In the biological information measurement method according to any one of the first to fifth aspects, the step of specifying the QRS interval may specify, as the QRS interval, a section from a certain time before the position of the peak of the R-wave to the position of the peak of the S-wave.

According to the biological information measurement method described in the sixth aspect, a situation in which the amplitude in the derivative electrocardiogram of the QRS interval is not accurately detected when the peak at the junction of the S-wave and the T-wave is large is reliably avoided.

(Seventh Aspect) The biological information measurement method according to any one of the first to sixth aspects may further comprise a step of performing noise removal of the electrocardiogram signal before the target region is specified.

According to the biological information measurement method described in the seventh aspect, the position of the R-wave peak and the position of the S-wave peak are specified from the electrocardiogram signal after noise removal, and thereby, the position of the R-wave peak and the position of the S-wave peak are specified more accurately.

(Eighth Aspect) The biological information measurement method according to any one of the first to sixth aspects may further comprise a step of estimating a state of the measurement subject based on the heart rate and the respiration rate.

According to the biological information measurement method described in the eighth aspect, the heart rate and the respiration rate are effectively used in providing information related to the measurement subject.

(Ninth Aspect) A program according to one aspect may, when executed by an electric circuit of an information processing device, cause the information processing device to perform the biological information measurement method according to any one of the first to eighth aspects.

According to the program described in the ninth aspect, a technique is provided for enabling the estimation of a respiration rate even when using an electrocardiogram waveform in which the R-wave peak is not detected with sufficient accuracy. Furthermore, according to the program described in the ninth aspect, a technique is provided for enabling the estimation of a respiration rate even when using an electrocardiogram waveform measured with an electrode placement in which the R-wave peak is not detected with sufficient accuracy.

(Tenth Aspect) An electrocardiograph according to one aspect may be an electrocardiograph comprising: a first electrode and a second electrode to be attached to a measurement subject; an electric circuit; and a memory, wherein the memory may, when executed by the electric circuit, cause the electrocardiograph to perform the biological information measurement method according to any one of the first to eighth aspects.

The embodiments disclosed this time should be considered illustrative in all respects and not restrictive. The scope of the present disclosure is indicated by the claims rather than by the description of the embodiments above, and is intended to include all modifications within the meaning and scope equivalent to the claims. Furthermore, it is intended that each technique in the embodiments can be implemented alone or in combination with other techniques in the embodiments as much as possible, as necessary.

### REFERENCE SIGNS LIST

1 main body device, 2 processing device, 11 holder, 12A, 12B electrode, 14 measurement circuit, 14A electrocardiogram signal detection unit, 14B transmission unit, 21 processor, 22 input/output port, 23 memory, 23A template data, 23B autoregressive model, 24 input device, 25 output device, 100 electrocardiograph, 230 data processing program, 231 noise removal unit, 232 division unit, 233 interval specification unit, 234 amplitude calculation unit, 235 heart rate calculation unit, 236 respiration rate calculation unit, 237 output unit.

## Claims

1. A biological information measurement method, comprising:
a step of acquiring an electrocardiogram signal from an output of a first electrode and a second electrode attached to a measurement subject;
a step of specifying, in each heartbeat of the electrocardiogram signal, a target region including a peak of an R-wave and a peak of an S-wave;
a step of specifying, in the target region of each heartbeat, a position of the peak of the S-wave;
a step of specifying, in the target region of each heartbeat, a position of a peak that is temporally prior to the position of the peak of the S-wave as a position of the peak of the R-wave;
a step of specifying a QRS interval within the target region of each heartbeat, using the position of the peak of the R-wave and the position of the peak of the S-wave;
a step of calculating a heart rate of the measurement subject by using intervals between the positions of the R-wave peaks specified in each heartbeat of the electrocardiogram signal; and
a step of calculating a respiration rate of the measurement subject by using a time series of amplitude in a derivative electrocardiogram of the QRS interval specified in each heartbeat of the electrocardiogram signal, and the heart rate.

2. The biological information measurement method according to claim 1, wherein
the first electrode is attached to an upper side of a sternum of the measurement subject, and
the second electrode is attached to a xiphoid process of the measurement subject.

3. The biological information measurement method according to claim 1, wherein the step of specifying the target region includes extracting the target region of each heartbeat by template matching with respect to the electrocardiogram signal.

4. The biological information measurement method according to claim 1, wherein the step of specifying the position of the peak of the S-wave includes:
obtaining a minimum value of a derivative waveform of the target region of each heartbeat;
obtaining a first maximum value in a section located after the minimum value in the derivative waveform of the target region of each heartbeat; and
extracting, as the position of the peak of the S-wave, a location where a value closest to 0 between the minimum value and the first maximum value is located in the derivative waveform of the target region of each heartbeat.

5. The biological information measurement method according to claim 4, wherein the step of specifying the position of the peak of the R-wave includes:
obtaining a second maximum value in a section located temporally prior to the minimum value in the derivative waveform of the target region of each heartbeat; and
extracting, as the position of the peak of the R-wave, a location where a value closest to 0 between the minimum value and the second maximum value is located in the derivative waveform of the target region of each heartbeat.

6. The biological information measurement method according to claim 1, wherein the step of specifying the QRS interval specifies, as the QRS interval, a section from a certain time before the position of the peak of the R-wave to the position of the peak of the S-wave.

7. The biological information measurement method according to claim 1, further comprising a step of performing noise removal of the electrocardiogram signal before the target region is specified.

8. The biological information measurement method according to claim 1, further comprising a step of estimating a state of the measurement subject based on the heart rate and the respiration rate.

9. A program which, when executed by an electric circuit of an information processing device, causes the information processing device to perform the biological information measurement method according to claim 1.

10. An electrocardiograph comprising:
a first electrode and a second electrode to be attached to a measurement subject;
an electric circuit; and
a memory, wherein
the memory, when executed by the electric circuit, causes the electrocardiograph to perform the biological information measurement method according to claim 1.
